# EUROPEAN PATENT SPECIFICATION

(11) **EP 3 073 991 B1**
(45) Date of publication and mention of the grant of the patent: **25.10.2017**
(21) Application number: 14806364.7
(22) Date of filing: 27.11.2014
(51) Int. Cl.: A61K 9/00, A61K 31/618, A61K 47/44, A61K 47/08, A61K 31/455, A61K 31/045, A61K 47/32, A61K 47/10

(54) **TOPICAL PHARMACEUTICAL COMPOSITION**
TOPISCHE PHARMAZEUTISCHE ZUSAMMENSETZUNG
COMPOSITION PHARMACEUTIQUE TOPIQUE

(30) Priority: 27.11.2013 GB 201320932
(43) Date of publication of application: 05.10.2016
(73) Proprietor: Futura Medical Developments Limited, The Surrey Research Park Guildford Surrey GU2 7YG (GB)
(72) Inventor: DAVIS, Adrian, Guildford Surrey GU2 7YG (GB)
(74) Representative: Wilson, Justin Scott
(86) International application number: PCT/GB2014/053510
(87) International publication number: WO 2015/079233

(56) References cited:
- WO-A2-2008/008474
- US-A- 4 892 890
- US-A1- 2012 213 717

## Description

This invention relates to topical pharmaceutical compositions. Particularly, it relates to such compositions for providing local relief from pain in underlying tissues, especially but not exclusively pain associated with muscle fatigue, stiff joints and arthritis.

Various topical analgesic compositions are commercially available in the form of gels, creams, sprays and patches, wherein the active ingredients comprise methyl salicylate and menthol. An example is Salonpas (RTM), in which the active ingredients are methyl salicylate and menthol (as the (-)-stereoisomer, levomenthol) in a ratio of 15:7 by weight. Other similar products are also commercially available; however, the pain relief provided by such products may have an unacceptably long onset time and/or an unacceptably short duration.

It is an object of the present invention to provide a topical analgesic composition comprising methyl salicylate, menthol and/or other similar active ingredients, and which is formulated to improve the analgesic effect, the onset time and/or the duration of the effect compared to known compositions comprising these actives.

In one aspect, the present invention provides a topical pharmaceutical composition comprising

| | |
|---|---|
| ester of salicylic or nicotinic acid | 10-20% |
| menthol | 5-10% |
| lower alcohol | 15-35% |
| water | 10-20% |
| propylene glycol and/or pentane-1,5-diol | 15-35% |
| hydrophilic pharmaceutically-acceptable non-volatile | |
| non-solvent for menthol | 2-16% |

and, optionally, up to 5% camphor,
all percentages being by weight.

It has surprisingly been found by the present inventor that, with known compositions including water, such as Salonpas, the sensory effect of menthol is lost once the water has evaporated from the skin following application, but may be restored by re-wetting the application site. This surprising finding led to the hypothesis that the transient presence of water increases the degree of saturation of the menthol, and hence the effect of menthol on the application site and adjacent tissues. The composition of the invention has been designed to exploit this finding, and to enhance both the degree of saturation and effects of the menthol, whilst also providing high percutaneous penetration of the salicylate or nicotinate ester. The presence of the hydrophilic non-solvent provides prolongation of the menthol enhancement effect, such that the menthol sensation is extended from 20-30 minutes in known compositions, to 1-2 hours or more in compositions of the invention. The result is a composition which provides a rapid menthol (and optionally camphor) sensation essentially as soon as the composition is applied, but which is longer lasting and allows for the sensation to be maintained throughout the period leading up to penetration of an effective amount of the salicylate or nicotinate ester.

When camphor is present, it may provide an additional rubefacient effect in conjunction with the menthol. In compositions of the invention, the menthol acts as a cosolvent for the camphor.

The ester of salicylic or nicotinic acid may be present in some embodiments at 12-20%, 12-18%, 14-16% or 15%. The menthol may, for example, be present at 6, 7, 8, or 9%. The propylene glycol and/or pentane-1,5-diol (preferably propylene glycol) is in particular embodiments present at 15-25%, or 15-20%. The lower alcohol may in particular be present at 20-35%, or 20-30%. The hydrophilic non-solvent may in particular be present at 2-10%, such as 5-10%.

Compositions according to the invention may also include minor amounts of additional excipients to improve the rheological properties or wetting characteristics, or to adjust the pH, preferably to between pH 6 and 7. For example, an acrylic acid polymer such as a crosslinked polyacrylate, e.g. a Carbopol (for example, a Carbopol Ultrez, such as Ultrez-10) may be added as a gelling agent (where the composition is to be in the form of a gel), and/or an organic amine such as diethylamine or triethanolamine, or an inorganic base such as NaOH or KOH may be used for pH adjustment. Fatty acids (and/or salts or esters (particularly glycerol esters, i.e. glycerides) thereof) may also be included, for example to enhance antiinflammatory, anti-fungal or other properties. Suitable fatty acids include C₁₀₋₂₀ unsaturated fatty acids, including blends or mixed glycerides thereof, optionally with saturated fatty acids of similar length. In particular, oleic, myristic, palmitic, palmitoleic, stearic, linoleic, and linolenic acids may be used. A suitable blend of fatty acids may, for example, be that which is present in Emu Oil (available from the Emu Oil Institute/Emu Products Ltd), containing glycerides comprising a preponderant amount of oleic acid and smaller amounts of the other fatty acids mentioned in this paragraph. Such fatty acid-containing ingredients may be present in an amount of up to 10% in total (based on the total weight of the composition). For example, from 2-10%, preferably 2-5%.

Compositions according to the invention are preferably in a single phase at room temperature (e.g. 15-30 degrees C, preferably 15-25 degrees C) at the time of application and may be in the form of a gel or spray (in the case of sprays, the composition may contain additional excipients necessary to achieve aerosolisation, such as a propellant (e.g. an HFC propellant)). In preferred embodiments, the ester of salicylic or nicotinic acid, the menthol (and optional camphor), the lower alcohol, the water, the propylene glycol and/or pentane-1,5-diol, and the hydrophilic pharmaceutically-acceptable non-volatile non-solvent for menthol are in a single phase at room temperature, whilst other ingredients, as discussed above (e.g. gelling agents, fatty acids/glycerides such as Emu Oil) may be dispersed throughout this single phase, even if they are not strictly in the same phase, as would be understood by the skilled person. The minimum concentrations of alcohol and water are thus those which will solubilise the ester of salicylic or nicotinic acid, the menthol (and optional camphor), the propylene glycol and/or pentane-1,5-diol, and the hydrophilic pharmaceutically-acceptable non-volatile non-solvent for menthol to ensure a single phase of these components overall. The relative concentrations of the components of the non-volatile residual phase are more significant in relation to the efficacy of the compositions.

Esters of salicylic acid which are suitable for use in compositions according to the invention include C₁₋₆ alkyl esters, such as the methyl, ethyl and propyl salicylates, preferably methyl salicylate. Esters of nicotinic acid include C₁₋₆ alkyl esters, such as the methyl and ethyl esters, and C₆₋₁₄ aralkyl (such as benzyl) esters. A preferred nicotinate ester is ethyl nicotinate.

By "lower alcohol" is meant an aliphatic alcohol having 1 to 5 carbon atoms. Such an alcohol is volatile in that it evaporates on application of the composition to the skin. The preferred alcohol is ethanol. Water also evaporates on application of the composition to the skin and for the purpose of the present invention is regarded as a volatile solvent. Propylene glycol and/or pentane-1,5-diol, on the other hand, is non-volatile in the context of the present invention. The hydrophilic non-volatile non-solvent is preferably a low molecular weight (e.g. less than 500 Da, preferably less than 200 Da, and more preferably less than 150Da) polyhydric alcohol, preferably glycerol. This constituent is also non-volatile and, together with the propylene glycol and/or pentane-1,5-diol, and the active ingredients (the ester and menthol (and optionally camphor)), comprise the residual phase on application of the composition to the skin.

Based on the composition according to the invention as hereinbefore described, the total non-volatile (residual) phase ranges from 32-81% by weight (excluding the optional presence of camphor, or other non-volatile ingredients) and the ranges of the non-volatile constituents expressed as percentages by weight of the total residual phase (excluding the optional presence of camphor, or other non-volatile ingredients) are as follows (to nearest whole percentage (actual figure in parentheses):

| | |
|---|---|
| ester of salicylic or nicotinic acid | 14 (14.1)-48 (47.6)% |
| menthol | 7 (6.6)-27 (27.0)% |
| propylene glycol and/or pentane-1,5-diol | 25 (24.6)-67 (67.3)% |
| hydrophilic pharmaceutically-acceptable non-volatile | |
| non-solvent for menthol | 3 (3.0)-35 (34.8)%. |

When camphor is present, these percentages by weight of the four main constituents of the non-volatile, residual phase are adjusted accordingly when expressed as % by weight of the total residual phase of the composition, as would be appreciated by the skilled person. Equally, if other non-volatile ingredients are included in the final composition, or incorporated into the non-volatile phase before mixture with the volatile components, these percentages by weight of the four main constituents of the non-volatile, residual phase are adjusted accordingly when expressed as % by weight of the total residual phase of the composition, as would be appreciated by the skilled person.

Thus, in another, related aspect, the invention provides a topical pharmaceutical composition comprising a volatile phase and a non-volatile (residual) phase, the non-volatile phase comprising from 32-81% of the total composition by weight, and comprising the following non-volatile constituents, expressed as % weight of the non-volatile phase:

| | |
|---|---|
| ester of salicylic or nicotinic acid | 14 (14.1)-48 (47.6)% |
| menthol | 7 (6.6)-27 (27.0)% |
| propylene glycol and/or pentane-1,5-diol | 25 (24.6)-67 (67.3)% |
| hydrophilic pharmaceutically-acceptable non-volatile | |
| non-solvent for menthol | 3 (3.0)-35 (34.8)%. |

The volatile phase may comprise water and/or a lower alcohol, and may comprise a blend of these components.

Moreover, in another related aspect, the invention provides a concentrate which may be used for the preparation of a topical pharmaceutical composition according to the invention, the concentrate comprising the components and amounts of the non-volatile phase defined in the aspect immediately above.

In compositions and concentrates according to the invention, the ratio of the salicylate or nicotinate ester to the menthol may, for example, be from 2.5:1 to 1:1 by weight, preferably from 2.5:1 to 2.0:1, for example 15:7 or 16:10. Clearly, at the 16:10 ratio there is a higher proportion of menthol. The menthol is present at an even higher proportion at a 1:1 ratio, although at this ratio the menthol is at saturation level in methyl salicylate, which has potential advantages for menthol's efficacy. At the higher proportions of menthol, higher hydrophilic non-solvent (e.g. glycerol) contents should be used, particularly at higher concentrations of propylene glycol and/or pentane-1,5-diol.

In compositions and concentrates according to the invention, the hydrophilic non-solvent content should be adapted, taking account the ester:menthol ratio and the propylene glycol and/or pentane-1,5-diol content, such that the menthol is at or just below its saturation point in the non-volatile system. The skilled person would readily be able to determine these levels, as illustrated in the Examples described herein.

The inclusion of propylene glycol and/or pentane-1,5-diol (propylene glycol is preferred), which is preferably present in a range of 17.5 to 22.5% by weight, for example 20% by weight, has the beneficial effect of enhancing skin penetration of the active ingredients. However, it also solubilises menthol and thus may reduce its degree of saturation and thus its thermodynamic activity in the residual phase. Nevertheless, the enhancement of skin penetration outweighs the solubilising effect, and the presence of propylene glycol and/or pentane-1,5-diol is thus beneficial. The hydrophilic pharmaceutically-acceptable non-volatile non-solvent acts as a persistent surrogate for the non-solvent volatile phase, whereby its presence in the residual phase maintains the sensory effect of the menthol (and optional camphor) even after evaporation of the water, thereby prolonging the duration of the analgesic and/or rubefacient effect.

It has been found that, whereas methyl salicylate, menthol (and, where present, camphor) and propylene glycol and/or pentane-1,5-diol are miscible in all proportions, the presence of glycerol causes, above certain concentrations, precipitation of the active ingredients. Therefore, for a given ratio of ester:menthol and concentration of propylene glycol and/or pentane-1,5-diol, the glycerol can be added at the optimum amount to cause near-saturation of the active ingredients, thus enhancing the sensory effect on application to the skin and evaporation of the volatile components.

The optional additional ingredients mentioned above are present as a dispersion throughout the volatile and non-volatile phases of the overall composition. When added to the non-volatile component during manufacture, the additional ingredients are dispersed throughout the non-volatile phase. When the volatile components are added, dispersion of the additional ingredients throughout the overall composition occurs.

In a further aspect, the present invention provides a composition of the invention as defined above, for use in therapy.

In a related aspect, the present invention provides a composition of the invention as defined above, for use in the treatment or prevention of pain.

Similarly, the present invention provides a composition of the invention for use in a method for treating or preventing pain in a subject in need thereof, the method comprising the topical application, to the skin of the subject, of a composition of the invention as defined above.

Embodiments of residual phases and topical compositions according to the invention (and, by extension, embodiments of concentrates according to the invention) will now be described by way of example only and with reference to the accompanying phase diagrams and Tables.

In the accompanying phase diagrams:
Figure 1 represents the ternary residual phase concentrations for a methyl salicylate:menthol ratio of 10:10 (1:1);
Figure 2 represents concentrations for a methyl salicylate:menthol ratio of 16:10 (1.6:1);
Figure 3 represents concentrations for a methyl salicylate:menthol ratio of 15:7 (2.14:1);
Figure 4 shows methyl salicylate skin permeation results in a number of compositions according to the invention, compared to known compositions (4a shows 24 hour results; 4b shows the same results but on a 6 hour scale);
Figure 5 shows the cumulative rate of methyl salicylate permeation across human skin at different time points after application of compositions according to the invention, compared to known compositions;
Figure 6 shows the percentage of applied dose of methyl salicylate permeated across human skin; and
Figure 7 shows cumulative methyl salicylate permeation across human skin using a number of compositions according to the invention, in comparison with Salonpas.

Referring to the phase diagrams, the plotted points represent, for each ratio of active ingredients, the situation in which to fixed amounts (2.2g) of the methyl salicylate:menthol blend are added varying amounts (from about 10% and up to about 40%) of propylene glycol, to provide clear solutions in each instance. Glycerol was then added dropwise until a slight haziness was observed, due to phase separation. Each individual plotted point thus shows the ternary percentage composition at which the haziness becomes apparent, and thus provides an indication of the phase separated, saturated, system. To the right of the lines of points, the composition (residual phase) is completely miscible; to the left of the lines, phase separation occurs.

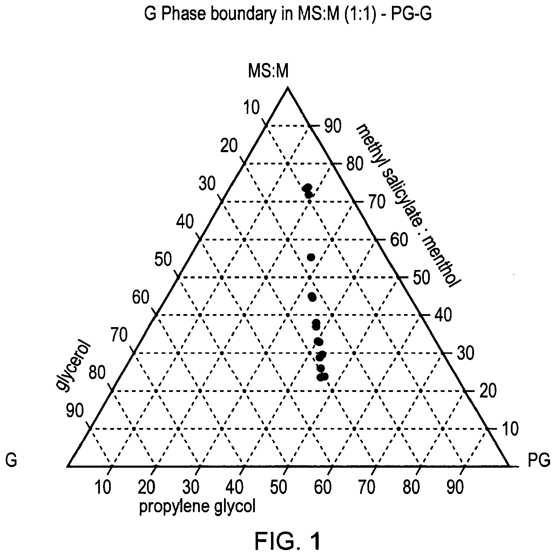

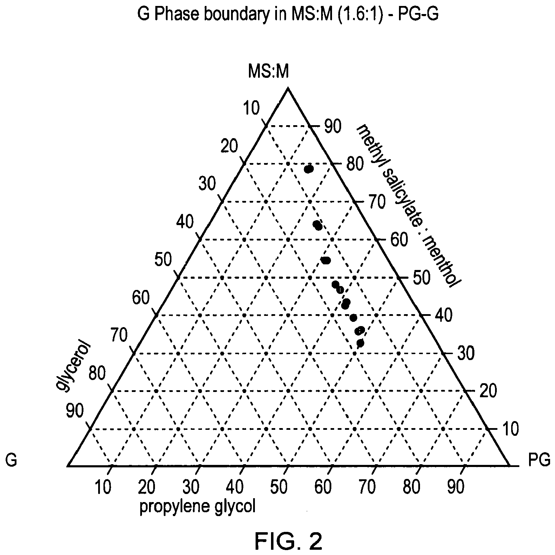

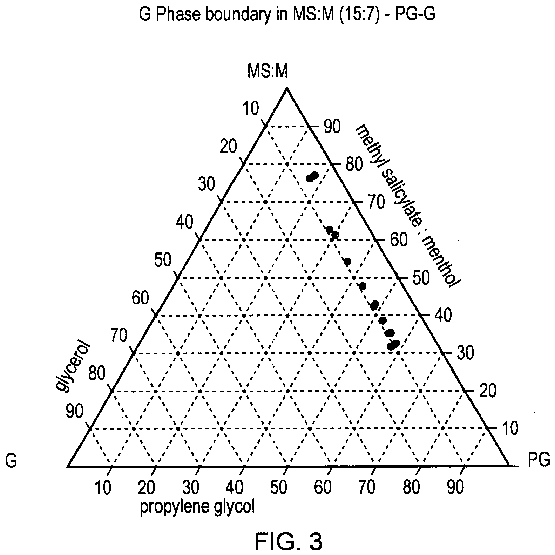

Thus, Fig.1 shows that, for a methyl salicylate:menthol ratio of 1:1, increasing the amount of propylene glycol requires increasing amounts of glycerol to achieve the limit of miscibility. Fig.2, showing the 1.6:1 ratio of methyl salicylate:menthol, shows a slight increasing requirement for glycerol with increasing propylene glycol, whereas Fig.3 shows that the glycerol requirement is consistently around 10% except at concentrations of propylene glycol greater than about 55%.

The data contained in Figures 1 to 3 is recited in Table 1, below. The final column indicates the amount of volatile phase (e.g. water and lower alcohol) which may be added to bring the composition to 100%.

**Table 1 - Phase boundary studies with methyl salicylate (MS):menthol (M) systems in propylene glycol (PG), with addition of glycerol (G)**

| MS:M ratio | MS:M % | PG % | G% at phase boundary | Approximate % volatile phase |
|---|---|---|---|---|
| 15:7 (2.14:1) | 22 | 25 | 4.6 | 48.4 |
| 15:7 (2.14:1) | 22 | 20 | 4.3 | 53.7 |
| 15:7 (2.14:1) | 22 | 15 | 3.8 | 59.2 |
| | | | | |
| 16:10 (1.6:1) | 26 | 25 | 9.3 | 39.7 |
| 16:10 (1.6:1) | 26 | 20 | 8.1 | 45.9 |
| 16:10 (1.6:1) | 26 | 15 | 6.6 | 52.4 |
| | | | | |
| 10:10 (1:1) | 20 | 25 | 16.3 | 38.7 |
| 10:10 (1:1) | 20 | 20 | 13.4 | 46.6 |
| 10:10 (1:1) | 20 | 15 | 9.94 | 55.06 |

Similar experiments were conducted with a system in which camphor was also included. The phase boundary results are reported in Table 2, below.

**Table 2 - Phase boundary studies with methyl salicylate (MS):menthol (M):camphor (C) systems in propylene glycol (PG), with addition of glycerol (G)**

| MS:M:C ratio | MS:M % | PG % | G% at phase boundary | Approximate % volatile phase |
|---|---|---|---|---|
| 15:7:4 | 26 | 25 | 6.87 | 42.13 |
| 15:7:4 | 26 | 20 | 6.10 | 47.9 |
| 15:7:4 | 26 | 15 | 5.36 | 53.64 |
| | | | | |
| 16:10:4 | 30 | 25 | 10.13 | 34.87 |
| 16:10:4 | 30 | 20 | 8.76 | 41.24 |
| 16:10:4 | 30 | 15 | 6.78 | 48.22 |
| | | | | |
| 10:10:4 | 24 | 25 | - | - |
| 10:10:4 | 24 | 20 | 12.16 | 43.84 |
| 10:10:4 | 24 | 15 | 9.25 | 51.75 |

It can be seen that, in a range of MS:M compositions also containing camphor (C), it is possible to bring menthol towards saturation at glycerol levels comfortably within the range defined for compositions of the invention.

Exemplary MS:M compositions according to the invention were prepared, based on the non-volatile phases reported in Table 1, with addition of volatile phase components and other optional ingredients, as shown in Table 3.

**Table 3 - Example formulations of MS:M topical pharmaceutical compositions according to the invention:**

| Ingredient | FM Gel 15:7 100g | FM Gel 16:10 100g | FM Gel 10:10 100g |
|---|---|---|---|
| Methyl salicylate | 15.00 | 16.00 | 10.00 |
| Menthol | 7.00 | 10.00 | 10.00 |
| | | | |
| Propylene glycol | 25.00 | 25.00 | 15.00 |
| Glycerol | 4.60 | 9.30 | 9.94 |
| | | | |
| Ethanol | 30.16 | 24.70 | 34.16 |
| Water | 16.74 | 13.50 | 19.90 |
| | | | |
| Carbopol Ultrez-10 | 1.00 | 1.00 | - |
| Diethylamine | 0.50 | 0.50 | - |
| HPC | - | - | 1.00 |
| Total | 100.00 | 100.00 | 100.00 |

Similarly, exemplary MS:M:C compositions according to the invention were prepared, based on the non-volatile phases reported in Table 2, with addition of volatile phase components and other optional ingredients, as shown in Table 4

**Table 4 - Example formulations of MS:M:C topical pharmaceutical compositions according to the invention**

| Ingredient | FM Gel 15:7:4 100g | FM Gel 16:10:4 100g | FM Gel 10:10:4 100g |
|---|---|---|---|
| Methyl salicylate | 15.00 | 16.00 | 10.00 |
| Menthol | 7.00 | 10.00 | 10.00 |
| Camphor | 4.00 | 4.00 | 4.00 |
| | | | |
| Propylene glycol | 20.00 | 20.00 | 15.00 |
| Glycerol | 6.10 | 8.76 | 9.25 |
| | | | |
| Ethanol | 30.16 | 26.24 | 32.85 |
| Water | 16.24 | 13.50 | 17.90 |
| | | | |
| Carbopol Ultrez-10 | 1.00 | 1.00 | - |
| Diethylamine | 0.50 | 0.50 | - |
| HPC | - | - | 1.00 |
| Total | 100.00 | 100.00 | 100.00 |

Furthermore, an exemplary formulation was prepared in which a fatty acid-containing ingredient (Emu Oil) was included, as shown in Table 5.

**Table 5 - Example formulation of MS:M:C topical pharmaceutical composition according to the invention, containing Emu Oil.**

| Ingredient | FM Gel 15:7#5:4 100g theoret to pH 6.00 |
|---|---|
| Methyl salicylate | 15 |
| Menthol | 7 |
| Camphor | 4 |
| Emu Oil | 3.5 |
| | |
| Ethanol | 25 |
| Water | 19 |
| | 0 |
| Propylene glycol | 20 |
| Glycerol | 5.5 |
| | |
| Carbopol Ultrez-10 | 1 |
| Diethylamine | to pH 6.00 |
| Total | 100 |

In each of the example formulations, it was possible to prepare the composition (i.e. including the volatile and non-volatile phases specified above) as a single phase system, in which the menthol was present in the non-volatile phase at or near saturation. Upon loss of the volatile components following use of the compositions, the menthol concentration moves towards saturation or supersaturation, and the compositions display enhanced menthol efficacy and enhanced methyl salicylate skin penetration.

It will be appreciated that in compositions according to the invention, the qualitative and quantitative design of the non-volatile phase is of primary importance. Provided the non-volatile phase is prepared such that the menthol is capable of saturation therein upon loss of the volatile components, the amounts and ratio of the volatile components can largely be chosen based on usability (i.e. appearance, feel, spreadability etc. in use) criteria.

A number of compositions according to the invention (and comparative compositions) were prepared and assessed in terms of their ability to delivery methyl salicylate (MS) transcutaneously. MS flux across human epidermal membranes (from three skin donors) was measured using a standard protocol (MedPharm, Ltd, Guildford, UK), and employing the following compositions:

| Ingredient | AD Gel 1a | AD Gel 2 | AD Spray 11 | AD Spray 12 |
|---|---|---|---|---|
| | %w/w | | | |
| Methyl salicylate | 15 | 15 | 10 | 15 |
| Menthol | 7 | 7 | 10 | 15 |
| Ethanol | 25.25 | 32 | 40 | 35 |
| Water | 18.75 | 12 | 20 | 15 |
| Propylene glycol | 33 | 33 | 18 | 18 |
| Glycerol | - | - | 2 | 2 |
| Carbopol Ultrez 10 | 0.75 | 0.75 | - | - |
| Diethylamine | 0.25 | 0.25 | - | - |
| Total | 100 | 100 | 100 | 100 |

AD Gels 1a and 2 are comparative compositions lacking glycerol. In addition, four commercial compositions were included in the test, as set out below:
- Salonpas (15% methyl salicylate, 7% menthol)
- Bengay (15% methyl salicylate, 10% menthol)
- Deepheat (12.8% methyl salicylate, 5.91% menthol)
- IcyHot (30% methyl salicylate, 10% menthol)

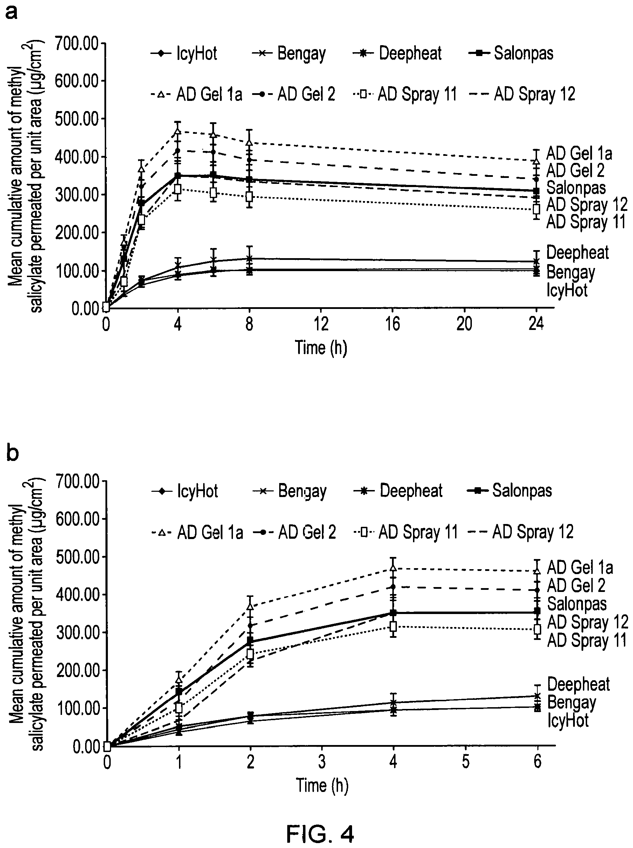

The results of the MS flux experiments are shown in Figures 4a and 4b. The results show that the MS:M 15:7 (including the commercial composition Salonpas) and 1:1 compositions provide similar, rapid and extensive delivery of MS through the skin. the second to fourth commercial compositions listed above all show poor PS delivery by comparison. Although the MS delivery of the compositions of the invention is demonstrated in these results to be of a similar level to Salonpas, the additional advantage of the compositions of the invention comes from the presence of glycerol, which is found to provide a much longer lasting menthol effect on the user.

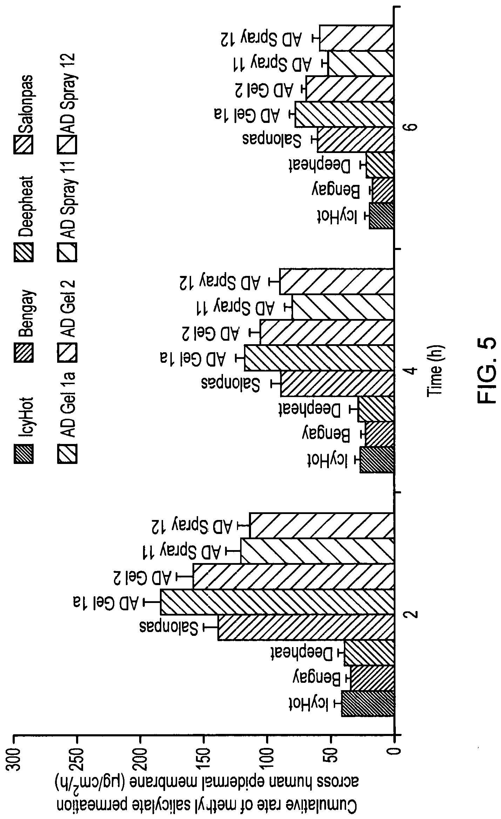

Figure 5 shows that for compositions of the invention, and Salonpas, the majority of the MS flux occurs in the first couple of hours following application. Compositions of the invention are therefore capable of delivering rapid pain relief, both due to the rapid delivery of MS, and to the rapid and long-lasting relief generated by the menthol.

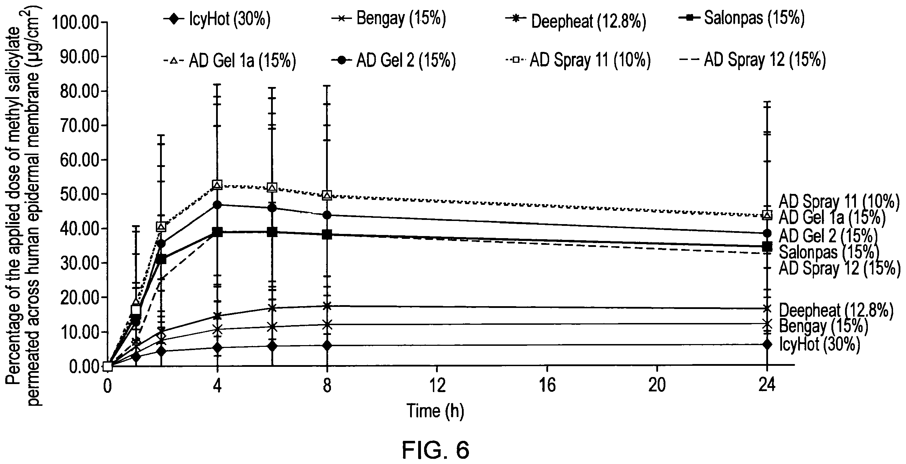

Figure 6 shows the MS absorption expressed as a percentage of applied dose. The results suggest a slight advantage of the composition AD Spray 11 (MS:M 1:1) in this test.

Further MS skin permeability tests were conducted with the following compositions according to the invention (AD Gel 1a included as comparison), and the commercial composition Salonpas listed above:

| Ingredient | AD gel 1a | AD gel 4 | AD gel 5 | AD Spray 12 |
|---|---|---|---|---|
| | %w/w | | | |
| Methyl salicylate | 15 | 16 | 15 | 15 |
| Menthol | 7 | 10 | 7 | 15 |
| Ethanol | 25.25 | 24 | 29.76 | 35 |
| Water | 18.75 | 13.5 | 16.74 | 15 |
| Propylene glycol | 33 | 25 | 25 | 18 |
| Glycerol | - | 10 | 5 | 2 |
| Carbopol Ultrez-10 | 0.75 | 1 | 1 | - |
| Diethylamine | 0.25 | 0.5 | 0.5 | - |
| Total | 100 | 100 | 100 | 100 |

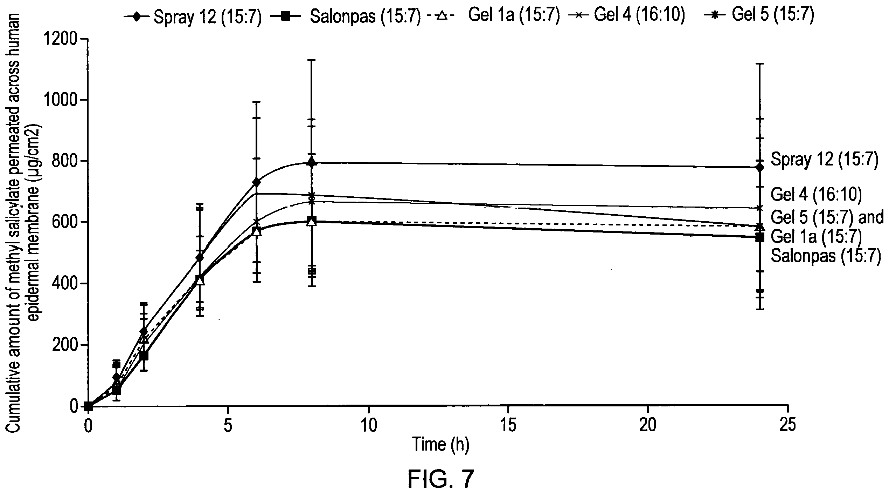

The results of MS flux (cumulative amount of MS permeated across human epidermal membrane) are shown in Figure 7. Again, the compositions of the invention show rapid and extensive delivery of MS, similar to the commercial Salonpas, but in each instance the compositions of the invention provide more rapid and extensive MS delivery. AD Spray 12 would appear to provide the most extensive MS delivery in this test. Again, the glycerol present in the compositions of the invention provide an enhancement in the efficacy of the composition, due to prolongation of menthol effects.

## Claims

1. A topical pharmaceutical composition comprising
| | |
|---|---|
| ester of salicylic or nicotinic acid | 10-20% |
| menthol | 5-10% |
| lower alcohol | 15-35% |
| water | 10-20% |
| propylene glycol and/or pentane-1,5-diol | 15-35% |
| hydrophilic pharmaceutically-acceptable non-volatile non-solvent for menthol | 2-16% |
and, optionally, up to 5% camphor,
all percentages being by weight.

2. A composition according to claim 1, wherein the ester of salicylic or nicotinic acid is present at 14-16%.

3. A composition according to claim 1 or claim 2, wherein the propylene glycol and/or pentane-1,5-diol is present at 15-25%.

4. A composition according to any preceding claim, wherein the hydrophilic non-solvent is present at 2-10%, such as 5-10%.

5. A composition according to any preceding claim, wherein the hydrophilic non-solvent comprises glycerol.

6. A composition according to any preceding claim, further including one or more additional excipients to improve rheological properties or wetting characteristics, or to adjust pH, wherein the composition optionally contains an acrylic acid polymer.

7. A composition according to claim 6, containing fatty acids, or salts or glycerides thereof, wherein the fatty acids are optionally present in the form of Emu Oil.

8. A composition according to any preceding claim, wherein the ester of salicylic acid is methyl salicylate, and/or wherein the ester of nicotinic acid is ethyl nicotinate.

9. A topical pharmaceutical composition comprising a volatile phase and a non-volatile phase, the non-volatile phase comprising from 32-81% of the total composition by weight, and comprising the following non-volatile constituents, expressed as % weight of the non-volatile phase:
| | |
|---|---|
| ester of salicylic or nicotinic acid | 14-48 % |
| menthol | 7-27 % |
| propylene glycol and/or pentane-1,5-diol | 25-67 % |
| hydrophilic pharmaceutically-acceptable non-volatile non-solvent for menthol | 3-35 %. |

10. A composition according to claim 9, wherein the volatile phase comprises water and/or a lower alcohol.

11. A concentrate which may be used for the preparation of a topical pharmaceutical composition according to any preceding claim, the concentrate comprising the following non-volatile constituents, expressed as % weight of the concentrate:
| | |
|---|---|
| ester of salicylic or nicotinic acid | 14-48 % |
| menthol | 7-27 % |
| propylene glycol and/or pentane-1,5-diol | 25-67 % |
| hydrophilic pharmaceutically-acceptable non-volatile non-solvent for menthol | 3-35 % |

12. A composition or concentrate according to any preceding claim, wherein the ratio of the salicylate or nicotinate ester to the menthol is from 2.5:1 to 1:1 by weight.

13. A composition according to any of claims 1 to 10 or 12, for use in therapy.

14. A composition according to any of claims 1 to 10 or 12, for use in the treatment or prevention of pain.

15. Use of a concentrate according to claim 11 for the manufacture of a topical pharmaceutical composition according to any of claims 1 to 10 or 12.

## Patentansprüche

1. Topische pharmazeutische Zusammensetzung, umfassend:
| | |
|---|---|
| Ester der Salicyl- oder Nikotinsäure | 10 - 20 % |
| Menthol | 5 - 10 % |
| niederer Alkohol | 15 - 35 % |
| Wasser | 10 - 20 % |
| Propylenglykol und/oder Pentan-1,5-diol | 15 - 35 % |
| hydrophiles pharmazeutisch akzeptables nichtflüchtiges Nichtlösungsmittel für Menthol | 2 - 16 % |
und optional bis zu 5 % Kampfer,
wobei alle Prozentangaben in Gewichtsprozent angegeben sind.

2. Zusammensetzung nach Anspruch 1, wobei der Ester der Salicyl- oder Nikotinsäure zu 14 - 16 % vorliegt.

3. Zusammensetzung nach Anspruch 1 oder 2, wobei das Propylenglykol und/oder Pentan-1,5-diol zu 15 - 25 % vorliegt.

4. Zusammensetzung nach einem der vorhergehenden Ansprüche, wobei das hydrophile Nichtlösungsmittel zu 2 - 10 % vorliegt, beispielsweise zu 5 - 10 %.

5. Zusammensetzung nach einem der vorhergehenden Ansprüche, wobei das hydrophile Nichtlösungsmittel Glycerin umfasst.

6. Zusammensetzung nach einem der vorhergehenden Ansprüche, welche ferner einen oder mehrere zusätzliche pharmazeutische Hilfsstoffe zur Verbesserung der rheologischen Eigenschaften oder Benetzungseigenschaften oder zur Einstellung des pH-Wertes umfasst, wobei die Zusammensetzung optional ein Acrylsäurepolymer enthält.

7. Zusammensetzung nach Anspruch 6, welche Fettsäuren oder Salze oder Glyceride dieser enthält, wobei die Fettsäuren optional in Form von Emuöl vorliegen.

8. Zusammensetzung nach einem der vorhergehenden Ansprüche, wobei der Ester der Salicylsäure Methylsalicylat ist, und/oder wobei der Ester der Nicotinsäure Ethylnicotinat ist.

9. Topische pharmazeutische Zusammensetzung, welche eine flüchtige Phase und eine nichtflüchtige Phase umfasst, wobei die nichtflüchtige Phase 32 - 81 % der Gesamtzusammensetzung in Gewichtsprozent ausmacht und wobei folgende nichtflüchtige Bestandteile umfasst sind, angegeben in Gewichtsprozent der nichtflüchtigen Phase:
| | |
|---|---|
| Ester der Salicyl- oder Nikotinsäure | 14 - 48 % |
| Menthol | 7 - 27 % |
| Propylenglykol und/oder Pentan-1,5-diol | 25 - 67 % |
| hydrophiles pharmazeutisch akzeptables nichtflüchtiges Nichtlösungsmittel für Menthol | 3 - 35 %. |

10. Zusammensetzung nach Anspruch 9, wobei die flüchtige Phase Wasser und/oder einen niederen Alkohol umfasst.

11. Konzentrat, das zur Herstellung einer topischen pharmazeutischen Zusammensetzung nach einem der vorhergehenden Ansprüche verwendet werden kann, wobei das Konzentrat die folgenden nichtflüchtigen Bestandteile umfasst, ausgedrückt in Gewichtsprozent des Konzentrats:
| | |
|---|---|
| Ester der Salicyl- oder Nikotinsäure | 14 - 48 % |
| Menthol | 7 - 27 % |
| Propylenglykol und/oder Pentan-1,5-diol | 25 - 67 % |
| hydrophiles pharmazeutisch akzeptables nichtflüchtiges Nichtlösungsmittel für Menthol | 3 - 35 %. |

12. Zusammensetzung oder Konzentrat nach einem der vorhergehenden Ansprüche, wobei das Verhältnis des Salicylat- oder Nicotinat-Esters zu Menthol 2,5:1 bis 1:1 bezogen auf das Gewicht beträgt.

13. Zusammensetzung nach einem der Ansprüche 1 bis 10 oder 12, zur Verwendung in der Therapie.

14. Zusammensetzung nach einem der Ansprüche 1 bis 10 oder 12, zur Verwendung bei der Behandlung oder Vorbeugung von Schmerzen.

15. Verwendung eines Konzentrats nach Anspruch 11 zur Herstellung einer topischen pharmazeutischen Zusammensetzung nach einem der Ansprüche 1 bis 10 oder 12.

## Revendications

1. Composition pharmaceutique à usage topique renfermant les composés suivants:
| | |
|---|---|
| ester d'acide salicylique ou d'acide nicotinique | 10-20% |
| menthol | 5-10% |
| alcool inférieur | 15-35% |
| eau | 10-20% |
| propylène glycol et/ou pentane 1,5 diol | 15-35% |
| composé hydrophile non volatile pharmaceutiquement acceptable ne constituant pas un solvant du menthol | 2-16% |
et, le cas échéant jusqu'à 5% de camphre,
tous les pourcentages étant des pourcentages pondéraux.

2. Composition conforme à la revendication 1, dans laquelle l'ester d'acide salicylique ou d'acide nicotinique est présent en proportion de 14-16%.

3. Composition conforme à la revendication 1 ou à la revendication 2, dans laquelle le propylène glycol et/ou le pentane 1,5-diol est présent en proportion de 15-25%.

4. Composition conforme à l'une quelconque des revendications précédentes dans laquelle le composé hydrophile ne constituant pas un solvant du menthol est présent en proportion de 2-10%, en particulier de 5-10%.

5. Composition conforme à l'une quelconque des revendications précédentes dans lequel le composé hydrophile ne constituant pas un solvant du menthol renferme du glycérol.

6. Composition conforme à l'une quelconque des revendications précédentes renfermant en outre au moins un excipient additionnel permettant d'améliorer les propriétés rhéologiques ou les caractéristiques de mouillage, ou de régler le pH, la composition renfermant le cas échéant un polymère d'acide acrylique.

7. Composition conforme à la revendication 6, renfermant des acides gras ou des sels ou glycérides de tels acides, les acides gras étant le cas échéant présents sous la forme d'huile d'émeu.

8. Composition conforme à l'une quelconque des revendications précédentes, dans laquelle l'ester d'acide salicylique est le méthyl salicylate et/ou l'ester d'acide nicotinique est l'éthyl nicotinate.

9. Composition pharmaceutique à usage topique renfermant une phase volatile et une phase non volatile, la phase non volatile constituant 32 à 81% en poids de la composition totale, et renfermant les composants non volatiles ci-dessous, exprimés en tant que proportion pondérale de la phase non volatile :
| | |
|---|---|
| ester d'acide salicylique ou d'acide nicotinique | 14-48% |
| menthol | 7-27% |
| propylène glycol et/ou pentane 1,5 diol | 25-67% |
| composé hydrophile non volatile pharmaceutiquement acceptable ne constituant pas un solvant du menthol | 3-35% |

10. Composition conforme à la revendication 9, dans laquelle la phase volatile renferme de l'eau et/ou un alcool inférieur.

11. Concentré susceptible d'être utilisé pour la préparation d'une composition pharmaceutique à usage topique conforme à l'une quelconque des revendications précédentes renfermant les composants non volatiles ci-dessous exprimés en pourcentage pondéral du concentré :
| | |
|---|---|
| ester d'acide salicylique ou d'acide nicotinique | 14-48% |
| menthol | 7-27% |
| propylène glycol et/ ou pentane-1,5 diol | 25-67% |
| composé hydrophile non volatile pharmaceutiquement acceptable ne constituant pas un solvant du menthol | 3-35% |

12. Composition ou concentré conforme à l'une quelconque des revendications précédentes dans lequel ou laquelle le rapport pondéral salicylate ester ou nicotinate ester / menthol est compris entre 2,5 :1 et 1 :1.

13. Composition conforme à l'une quelconque des revendications 1 à 10 ou 12, destinée à être utilisée en thérapie.

14. Composition conforme à l'une quelconque des revendications 1 à 10 ou 12, destinée à être utilisée pour le traitement ou la prévention de la douleur.

15. Utilisation d'un concentré conforme à la revendication 11, pour la préparation d'une composition pharmaceutique à usage topique conforme à l'une quelconque des revendications 1 à 10 ou 12.
